# EUROPÄISCHE PATENTSCHRIFT

(11) **EP 0 858 283 B1**
(45) Veröffentlichungstag und Bekanntmachung des Hinweises auf die Patenterteilung: **09.01.2002**
(21) Anmeldenummer: 96945513.8
(22) Anmeldetag: 18.10.1996
(51) Int. Cl.: A61B 1/31

(54) **REKTOSKOP**
RECTOSCOPE
RECTOSCOPE

(30) Priorität: 19.10.1995 DE 29516561 U
(43) Veröffentlichungstag der Anmeldung: 19.08.1998
(73) Patentinhaber: Karl Storz GmbH & Co. KG, 78532 Tuttlingen (DE)
(72) Erfinder: STORZ, Karl, DECEASED (DE)
(74) Vertreter: Münich, Wilhelm, Dr.
(86) Internationale Anmeldenummer: DE9601989
(87) Internationale Veröffentlichungsnummer: WO9714287

(56) Entgegenhaltungen:
- FR-A- 979 425
- GB-A- 203 784
- US-A- 3 889 661
- US-A- 4 306 546

## Beschreibung

### Technisches Gebiet

Die Erfindung bezieht sich auf ein Rektoskop zur Untersuchung insbesondere des Enddarmes gemäß dem Oberbegriff des Patentanspruchs 1.

### Stand der Technik

Gattungsgemäße Rektoskope werden beispielsweise von der Karl Storz GmbH & Co. KG, Tuttlingen, Deutschland hergestellt und weisen ein weitlumiges Rohr auf, dessen distaler Bereich z.B. in den Enddarm einsetzbar ist.

Ferner ist eine Beleuchtungseinrichtung vorhanden, deren Licht proximal- oder distalseitig in das weitlumige Rohr eingekoppelt wird. Die Untersuchungsperson betrachtet den zu untersuchenden Bereich des Enddarms durch das offene proximale Ende des weitlumigen Rohrs ohne zusätzliche Betrachtungshilfen, wie ein Mikroskop oder dgl..

Die bekannten gattungsgemäßen Rektoskope haben damit den Nachteil, daß die Betrachtung des zu untersuchenden Bereichs des Enddarms mit dem bloßen Auge erfolgt. Damit ist die Untersuchungsperson ungeschützt beispielsweise gegenüber Blutspritzern. Vor allem aber liefert die Betrachtung insbesondere dann, wenn die Untersuchungsperson ein Brillenträger ist, kein kontrastreiches Bild, da die Reflexionen des Beleuchtungslichts an der Innenwand des weitlumign Rohres störend sind.

Zur Lösung der zuvor beschriebenen Probleme bietet die US-A-3,889,661 ein auch als Rektoskop verwendbares Instrument an, das einen Lichtleiteranschluss aufweist, mit dem das Licht einer Beleuchtungseinrichtung in ein Rohr eingekoppelbar ist, und einer Betrachtungsoptik, wobei die Innenseite des Rohrlumens behandelt ist, um als Lichtleiter für das Licht der Beleuchtungseinrichtung zu dienen. Das Instrument besitzt
- ein Rohr, dessen distaler Bereich in den Körper einsetzbar ist,
- einen Lichtleiteranschluß, mittels dem das Licht einer Beleuchtungseinrichtung in das Rohr eingekoppelt wird, und
- eine Betrachtungsoptik, die im proximalen Bereich des Rohrs angeordnet ist.

Diese Ausführung gewährleistet jedoch nicht, dass ein kontrastreiches Bild des zu untersuchenden Bereichs zur Verfügung gestellt wird. Die Ursache dafür liegt darin, dass die Reflexionen des Beleuchtungslichtes an der Innenwand des Rohres stören, da sie im Sichtfeld des Betrachters liegen.

### Darstellung der Erfindung

Der Erfindung liegt die Aufgabe zugrunde, ein Rektoskop gemäß dem Oberbegriff des Anspruchs 1 derart weiterzubilden, daß ein kontrastreiches Bild des zu untersuchenden Bereichs zur Verfügung gestellt wird, ohne daß eine distalseitige Optik eingesetzt werden muß, und ohne daß Reflexionen des Betrachtungslichtes an der Innenwand des Rohres das Sichtfeld des Betrachters stören.

Eine erfindungsgemäße Lösung dieser Aufgabe ist im Patentanspruch 1 angegeben. Weiterbildungen der Erfindung sind Gegenstand der Ansprüche 2 folgende.

Erfindungsgemäß ist die am proximalen Ende des weitlumigen Rohrs angebrachte Betrachtungsoptik, die auf den zentralen Bereich der distalen Öffnung des Rohrs fokussierbar ist, als Endoskop ausgeführt, dessen Länge so bemessen ist, daß es nur zum Teil in das weitlumige Rohr hineinragt, so daß das Endoskopobjektiv (noch) im proximalen Bereich angeordnet ist. Damit kann die Betrachtungsoptik in einfacher und damit kostengünstiger Weise aus für andere Zwecke bereits vorhandenen Bestandteilen von Endoskopen aufgebaut werden. Diese Betrachtungsoptik, deren Fokusebene im Bereich der distalen Öffnung liegt, liefert ein kontrastreiches Bild, da die Reflexionen des Beleuchtungslichtes an der teilreflektierenden Innenwand des Rohres für den Betrachter aufgrund der Fokussierung auf die Öffnung nicht mehr stören. Dabei ist es von besonderem Vorteil, wenn die Vergrößerung der Betrachtungsoptik so gewählt ist, daß der Betrachter lediglich die distale Öffnung und gegebenenfalls einen kleinen Teil des distalen Rohrendes sieht.

Bei einer bevorzugten Weiterbildung ist die Fokusebene der Betrachtungsoptik einstellbar. Damit ist es möglich, auch Bereiche außerhalb der Ebene, in der sich die distale Öffnung des Rektoskops befindet, scharf zu betrachten.

Als Betrachtungsoptiken können im Prinzip beliebige Optiken verwendet werden, solange diese nur in der Lage sind, bei einem im Bereich des proximalen Endes des Rektoskops angeordneten Abschlußfenster der Betrachtüngsoptik die Beobachtung bzw. Betrachtung des distalen Endes des Rektoskops zu ermöglichen.

Weiterhin ist es ist auch in einfacher Weise möglich, den Strahlengang abzuwinkeln, so daß das mit der Rohrachse einen Winkel einschließende Okular den freien Zugang zum proximalen Ende des Rektoskops nicht behindert.

Der freie Zugang zum proximalen Ende wird weiter dadurch erleichtert, daß die Betrachtungsoptik bezogen auf das weitlumige Rohr außermittig angeordnet ist.

Insbesondere kann die Betrachtungsoptik über eine Standardkupplung an einem Flansch angebracht sein. Dieses Instrument kann aufgrund der Tatsache, daß sich sowohl die Betrachtungsoptik als auch die Beleuchtungseinrichtung im proximalen Bereich des weitlumigen Rohres befinden, ungehindert über den gesamten Bereich der distalen Öffnung des Rohres bewegt und gleichzeitig mit der Betrachtungsoptik beobachtet werden.

Zur Vermeidung von Reflexionen ist es weiter von Vorteil, wenn die Achsen der Beleuchtungseinrichtung und der Betrachtungsoptik einen Winkel <> 0° einschlie0en.

### Kurze Beschreibung der Zeichnung

Die Erfindung wird nachstehend ohne Beschränkung des allgemeinen Erfindungsgedankens anhand eines Ausführungsbeispiels unter Bezugnahme auf die Zeichnung exemplarisch beschrieben,
deren einzige Figur einen Querschnitt durch ein erfindungsgemäßes Rektoskop zeigt.

### Darstellung eines Ausführungsbeispiels

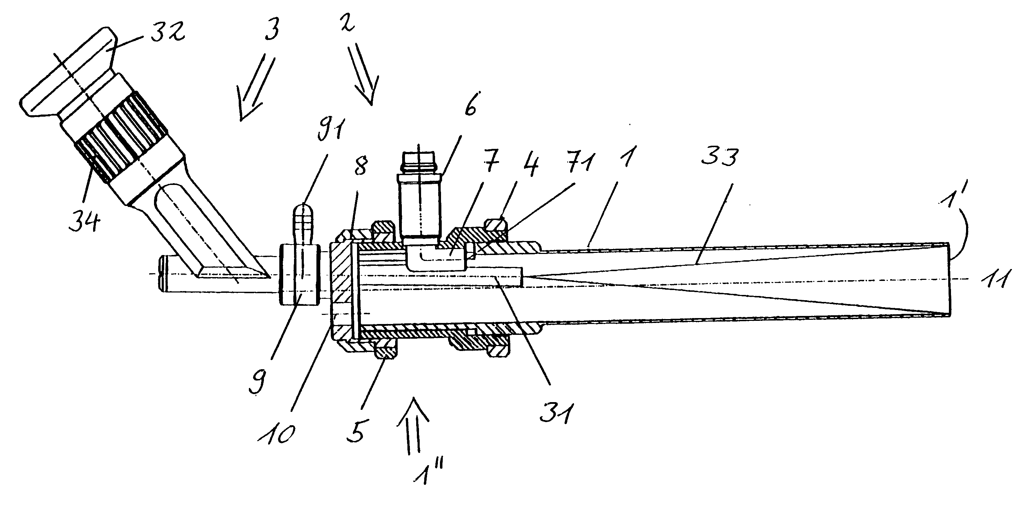

Das in Fig. 1 dargestellte Rektoskop, das insbesondere zur Untersuchung des Enddarmes geeignet ist, weist ein weitlumiges Rohr 1 auf, das an seinem distales Ende 1', also vorne offen ist. Das Rektoskop wird mit seinem distalen Ende 1' beispielsweise in den Enddarm eingesetzt.

Erfindungsgemäß sind im Bereich des proximalen Endes 1" des weitlumigen Rohrs 1 eine Beleuchtungseinrichtung 2 und eine Betrachtungsoptik 3 angeordnet.

Hierzu sind an dem Rohr 1 zwei Kupplungen 4 bzw. 5 vorgesehen, von denen die Kupplung 4 die lösbare Verbindung der Beleuchtungseinrichtung 2 und die Kupplung 5 die lösbare Verbindung der Betrachtungsoptik 3 mit dem Rohr 1 gestattet.

Die Beleuchtungseinrichtung 2 weist einen Lichtleiteranschluß 6 und ein Lichtleiterelement 7 auf, das durch eine Öffnung in dem Rohr 1 in dessen Inneres hineinragt.

Der Lichtleiteranschluß 6, der in gleicher Weise wie bei bekannten Endoskopen beispielsweise der Karl Storz GmbH & Co. ausgeführt sein kann, befindet sich außerhalb des Rohres 1 und erlaubt das Einkoppeln des Lichts einer nicht dargestellten Beleuchtungslichtquelle über ein ebenfalls nicht dargestelltes Lichtleiterkabel. Die Verbindung einer herkömmlichen Beleuchtungslichtquelle über ein Lichtleitkabel mit dem Lichtleiteranschluß 6 ist bekannt und muß daher an dieser Stelle nicht näher erläutert werden.

Das Lichtleiterelement 7 leitet das in den Lichtleiteranschluß 6 eingekoppelte Licht zu seiner in dem Rohr angeordneten Lichtaustrittsfläche 71, die sich bevorzugt - wie bei dem gezeigten Ausführungsbeispiel - im Bereich des proximalen Endes 1" des Rohres 1 befindet. Das aus der Lichtaustrittsfläche 71 des Lichtleiterelements 7 austretende Licht wird in das weitlumige Rohr eingekoppelt.

Bei dem gezeigten Ausführungsbeispiel ist der in das Innere des Rohrs 1 ragende Teil des Lichtleiterelements 7 nicht in der Rohrachse 11 angeordnet. Die Innenseite des Rohrs 1 ist teilreflektierend ausgebildet ist, so daß die Innenseite des Rohrs als Licht-Weiterleiter für das aus der Lichtaustrittsfläche 71 austretende Licht dient. Damit beleuchtet das aus der Lichtaustrittsfläche 71 austretende Licht - gegebenenfalls nach Reflexion an der Innenwand des Rohres 1 - die distale Endfläche 1' sowie den sich hieran anschließenden Bereich des Körperinneren.

Ferner ist es möglich, daß die Beleuchtungseinrichtung 2 an ihrer Lichteintritts- oder an ihrer Lichtaustrittsseite ein (in der Zeichnung nicht dargestelltes) optisches System aufweist, das eine Fokussierung bzw. eine Änderung des Öffnungswinkels des aus der Lichtaustrittsfläche 71 austretenden Lichtkegels erlaubt, so daß die Größe des beleuchteten Körperbereichs variiert werden kann.

Bei dem dargestellten Ausführungsbeispiel ist ein Flansch 8 vorgesehen, der in Art eines Deckels die proximale Öffnung des Rohres 1 abdeckt und der mittels der Kupplung 5 lösbar fest mit dem Rohr 1 verbunden ist. Der Flansch 8 trägt eine Kupplung 9, die in Art einer herkömmlichen Endoskop-Kupplung ausgebildet ist, wie sie beispielsweise an Trokarschäften zum Ankuppeln von Endoskopen vorhanden ist. Mit 91 ist ein Hebel zum Bedienen der Kupplung 9 bezeichnet.

Mittels der Kupplung 9 ist mit dem Flansch 8 ein Endoskop verbunden, das als Betrachtungsoptik 3 dient. Bei dem gezeigten Ausführungsbeispiel weist das Endoskop 3 einen stabförmigen Teil 31 und einen Okularteil 32 auf, der zur Verbesserung der Zugänglichkeit und zur Vereinfachung der Handhabung gegenüber dem stabförmigen Teil 31 abgewinkelt ist.

Die Länge des stabförmigen Teils 31 ist so bemessen, daß dieser nur zum Teil in das Rohr 1 hineinragt. Das in der Zeichnung nicht dargestelle Endoskopobjektiv hat einen Öffnungswinkel 33, der derart gewählt ist, daß die distale Endfläche 1' des Rohrs 1 vollständig abgebildet wird.

Das Bild des Endoskopobjektivs wird in an sich bekannter Weise von einem Bildweiterleiter, der beispielsweise Stablinsen-Relaissysteme oder ein Abbildungsfaserbündel aufweisen kann, zum proximalen Ende weitergeleitet, so daß es mittels des Okulars betrachtet werden kann.

Weiterhin ist die Betrachtungsoptik 3 so ausgebildet, daß sie auf den zentralen Bereich der distalen Öffnung 1' des Rohrs 1 fokussierbar ist; dabei ist die Fokusebene der Betrachtungsoptik 3 einstellbar. Zur Fokussierung dient ein Einstellring 34.

Wie die Zeichnung zeigt, ist der stabförmige Teil 31 des als Betrachtungsoptik 3 dienenden Endoskops ebenfalls außerhalb der Rohrachse 11 angeordnet und schließt mit dieser einen Winkel <> 0° ein.

Weiterhin weist der Flansch 8 einen Kanal 10 für den Einsatz eines weiteren Instruments auf. Da sowohl die Beleuchtungseinrichtung 2 als auch die Betrachtungsoptik 3 im proximalen Bereich 1" des Rohrs 1 außerhalb der Rohrachse

Vorstehend ist die Erfindung anhand eines Ausführungsbeispiels ohne Beschränkung des allgemeinen Erfindungsgedankens, wie er insbesondere den Ansprüchen zu entnehmen ist, beschrieben worden. Innerhalb dieses allgemeinen Erfindungsgedankens sind selbstverständlich die verschiedensten Modifikationen möglich:

So ist es insbesondere möglich, die Beleuchtungseinrichtung und die Betrachtungsoptik zu einer Einheit zusammenzufassen, wie dies bei Endoskopen allgemein üblich ist, bei denen sowohl die Beleuchtungs-Lichtleiter als auch die Relaislinsensysteme in ein- und demselben Roh verlaufen.

## Patentansprüche

1. Rektoskop zur Untersuchung beispielsweise des Enddarmes mit
- einem Rohr (1), dessen distaler Bereich (1') in den Körper einsetzbar ist,
- einem Lichtleiteranschluß (6), mittels dem das Licht einer Beleuchtungseinrichtung in das Rohr (1) eingekoppelt wird, und
- einer Betrachtungsoptik (3) , die im proximalen Bereich (1") des Rohrs (1) angeordnet ist ,
**dadurch gekennzeichnet, daß** die Betrachtungsoptik ein Endoskop (3) ist, dessen Länge so bemessen ist, daß es nur zum Teil in das Rohr (1) hineinragt, daß die Betrachtungsoptik (3) auf den zentralen Bereich der distalen Öffnung (1') des Rohrs (1) fokussierbar ist, und daß die Innenseite, des Rohrs (1) teilreflektierend ausgebildet ist, so daß die Innenseite des Rohrs (1) als Lichtleiter für das Licht der Beleuchtungseinrichtung derart dient, daß ein kontrastreiches Bild des zu untersuchenden Bereichs zur Verfügung gestellt wird.

2. Rektoskop nach Anspruch 1,
**dadurch gekennzeichnet, daß** die Fokusebene der Betrachtungsoptik (3) einstellbar ist.

3. Rektoskop nach Anspruch 1 oder 2,
**dadurch gekennzeichnet, daß** das Okular (32) des Endoskops (3) einen Winkel <> 0° mit dem stabförmigen Teil (31) einschließt, der in das Rohr (1) eingesetzt ist.

4. Rektoskop nach Anspruch 1 bis 3,
**dadurch gekennzeichnet, daß** sich das Objektiv des Endoskops (3) im proximalen Bereich (1") des Rohrs (1) befindet.

5. Rektoskop nach einem der Ansprüche 1 bis 4,
**dadurch gekennzeichnet, daß** die Betrachtungsoptik (3) bezogen auf das Rohr (1) außermittig angeordnet ist.

6. Rektoskop nach einem der Ansprüche 1 bis 5,
**dadurch gekennzeichnet, daß** ein Flansch (8) vorgesehen ist, der mit dem Rohr (1) mittels einer Kupplung (5) verbindbar ist, und an dem die Betrachtungsoptik (3) angebracht ist.

7. Rektoskop nach Anspruch 6,
**dadurch gekennzeichnet, daß** die Betrachtungsoptik (3) mit dem Flansch (8) mittels einer Standard-Endoskopkupplung (9) verbunden ist.

8. Rektoskop nach Anspruch 6 oder 7,
**dadurch gekennzeichnet, daß** der Flansch (8) wenigstens einen Kanal (10) für den Einsatz eines weiteren Instruments aufweist.

9. Rektoskop nach einem der Ansprüche 1 bis 8,
**dadurch gekennzeichnet, daß** die Lichtaustrittsfläche (71) der Beleuchtungseinrichtung (2) im proximalen Bereich (1") des Rohres (1) angeordnet ist.

10. Rektoskop nach Anspruch 9,
**dadurch gekennzeichnet, daß** die Lichtaustrittsfläche (71) nicht in der Achse (11) des Rohrs (1) angeordnet ist.

11. Rektoskop nach einem der Ansprüche 1 bis 10,
**dadurch gekennzeichnet, daß** die Achsen der Beleuchtungseinrichtung (2) und der Betrachtungsoptik (3) einen Winkel <> 0° einschließen.

## Claims

1. Rectoscope for examining the rectum, for instance, comprising
- a tube (1) whose distal region (1') is adapted for being introduced into the body,
- a fibre-optical light guide connector (6) by means of which the light of an illuminating means (1) is introduced into said tube (1),
- an optical system (3) disposed in the proximal zone (1") of said tube (1),
**characterised in that** said optical observation system is an endoscope (3) having a length so dimensioned that the endoscope projects into said tube (1) only partly, that said optical observation system (3) is adapted for being focussed on the central region of the distal opening (1') of said tube (1), and that the inside surface of said tube (1) is designed for partial reflection so that the inside surface of said tube (1) serves as light guide for the light of said illuminating means so as to provide a high-contrast image of the region to be examined.

2. Rectoscope according to Claim 1,
**characterised in that** the focal plane of said optical observation system (3) is adjustable.

3. Rectoscope according to Claim 1 or 2,
**characterised in that** the eyepiece (32) of the endoscope (3) forms an angle of <> 0° relative to the rod-shaped element (321) that is introduced into said tube (1).

4. Rectoscope according to the Claims 1 to 3,
**characterised in that** the lens of the endoscope is located in the proximal zone (1") of said tube (1).

5. Rectoscope according to any of the Claims 1 to 4,
**characterised in that** said optical observation system (3) is arranged in an eccentric position relative to said tube (1).

6. Rectoscope according to any of the Claims 1 to 5,
**characterised in that** a flange (8) is provided that is adapted to be connected to said tube (1) by means of a coupler (5) and on which said optical observation system (3) is mounted.

7. Rectoscope according to Claim 6,
**characterised in that** said optical observation system (3) is connected to said flange (8) by means of a standard endoscope coupler (9).

8. Rectoscope according to Claim 6 or 7,
**characterised in that** said flange (8) comprises at least one passage (10) for the introduction of a further instrument.

9. Rectoscope according to any of the Claims 1 to 8,
**characterised in that** the light exit surface (71) of said illuminating means (2) is disposed in the proximal zone (1") of said tube (1).

10. Rectoscope according to Claim 9,
**characterised in that** said light exit surface (71) is not located in the axis (11) of said tube (1).

11. Rectoscope according to any of the Claims 1 to 10,
**characterised in that** the axes of said illuminating means (2) and of said optical observation system (3) enclose an angle of <> 0°.

## Revendications

1. Rectoscope à examiner le rectum, par exemple, comprenant
- un tube (1), dont la zone distale (1') est apte à être introduite dans le corps,
- un raccord (6) pour guide de lumière, moyennant lequel la lumière d'un moyen d'éclairage (1) est introduite dans ledit tube (1),
- un système optique (3) disposé dans la zone proximale (1") dudit tube (1),
**caractérisé en ce que** ledit système optique d'observation est un endoscope (3) à une longueur dimensionnée de façon, que l'endoscope ne projette dans ledit tube (1) qu'en partie, **en ce que** ledit système optique d'observation (3) est apte à être focalisé sur la zone centrale de l'ouverture distale (1') dudit tube (1), et **en ce que** la face intérieure dudit tube (1) est formée à créer des réflexions partielles de façon, que la face intérieure dudit tube (1) sert en tant que guide de lumière pour la lumière dudit moyen d'éclairage d'une manière à fournir une image à haut contraste de la zone à examiner.

2. Rectoscope selon la revendication 1,
**caractérisé en ce que** le plan focal dudit système optique d'observation (3) est ajustable.

3. Rectoscope selon la revendication 1 ou 2,
**caractérisé en ce que** l'oculaire (32) de l'endoscope (3) forme un angle de <> 0° relatif à la pièce sous forme de bâtonnet (321), qui est introduite dans ledit tube (1).

4. Rectoscope selon les revendications 1 à 3,
**caractérisé en ce que** l'objectif de l'endoscope se trouve dans la zone proximale (1") dudit tube (1).

5. Rectoscope selon une quelconque des revendications 1 à 4,
**caractérisé en ce que** ledit système optique d'observation (3) est disposé de façon excentrique relativement audit tube (1).

6. Rectoscope selon une quelconque des revendications 1 à 5,
**caractérisé en ce qu'**une collerette (8) est disposée, qui est apte à être raccordée audit tube (1) moyennant un coupleur (5) et sur lequel ledit système optique d'observation (3) est monté.

7. Rectoscope selon la revendication 6,
**caractérisé en ce que** ledit système optique d'observation (3) est raccordé à ladite collerette (8) moyennant un coupleur d'endoscope standard (9).

8. Rectoscope selon la revendication 6 ou 7,
**caractérisé en ce que** ladite collerette (8) comprend au moins un passage (10) pour l'introduction d'un autre instrument.

9. Rectoscope selon une quelconque des revendications 1 à 8,
**caractérisé en ce que** la face de sortie de lumière (71) dudit moyen d'éclairage (2) est disposée dans la zone proximale (1") dudit tube (1).

10. Rectoscope selon la revendication 9,
**caractérisé en ce que** ladite face de sortie de lumière (71) n'est pas disposée dans l'axe (11) dudit tube (1).

11. Rectoscope selon une quelconque des revendications 1 à 10,
**caractérisé en ce que** les axes dudit moyen d'éclairage (2) et dudit système optique d'observation (3) forment un angle de <> 0°.
